# EUROPEAN PATENT SPECIFICATION

(11) **EP 2 911 712 B1**
(45) Date of publication and mention of the grant of the patent: **29.11.2017**
(21) Application number: 13759401.6
(22) Date of filing: 28.08.2013
(51) Int. Cl.: A61L 31/10, A61L 31/14

(54) **STENT HAVING A TACKY SILICONE COATING TO PREVENT STENT MIGRATION**
STENT MIT EINER KLEBRIGEN SILIKONBESCHICHTUNG ZUR MIGRATIONSVERHINDERUNG
ENDOPROTHÈSE DOTÉ D'UN REVÊTEMENT DE SILICONE COLLANT POUR EMPÊCHER UNE MIGRATION D'ENDOPROTHÈSE

(30) Priority: 25.10.2012 US 201261718288 P
(43) Date of publication of application: 02.09.2015
(73) Proprietor: Boston Scientific Scimed, Inc., Maple Grove, MN 55311 (US)
(72) Inventor: CLERC, Claude O., Marlborough, Massachusetts 01752 (US); FREDRICKSON, Gerald, Westford, Massachusetts 01886 (US); ROOT, Jonathan, Groveland, Massachusetts 01834 (US)
(74) Representative: Vossius & Partner Patentanwälte Rechtsanwälte mbB
(86) International application number: PCT/US2013/057063
(87) International publication number: WO 2014/065941

(56) References cited:
- WO-A1-00/40278
- WO-A1-2006/038866
- US-B1- 6 251 136

## Description

### BACKGROUND OF THE INVENTION

The present invention relates generally to coated medical devices, in particular, to covered stents and to methods of using a tacky polymeric material in a patient's body lumen to prevent stent migration from a treatment site.

Stents, grafts, stent-grafts, vena cava filters and similar implantable medical devices, collectively referred to hereinafter as stents, are radially expandable or self-expanding endoprostheses which are intravascular or endoscopic implants capable of being implanted transluminally either percutaneously or endoscopilcally. Stents may be implanted in a variety of body lumens or vessels such as within the vascular system, urinary tracts, bile ducts, gastro-intestinal tract, airways, etc. Stents may be used to reinforce body vessels and to prevent restenosis following angioplasty in the vascular system. They may be self-expanding, mechanically expandable or hybrid expandable. In general, self-expanding stents are mounted on a delivery device consisting of two tubes. The stent is delivered by sliding the outer tube to release the stent.

Stents are typically tubular members that are radially expandable from a reduced diameter configuration for delivery through a patient's body lumen to an expanded configuration once deployed at the treatment site.

Stents may be constructed from a variety of materials such as stainless steel, Elgiloy, nickel, titanium, nitinol, polymers, shape memory polymers, etc.

Typically, the stent is formed from a tubular member in which a pattern is subsequently formed by etching or cutting material from the tubular member or it is made from wires using techniques such as braiding, knitting or weaving

Desirable stent properties thus include sufficient flexibility to be able to conform to the tortuous body lumen during delivery, yet sufficiently rigid to resist migration once deployed at the treatment site.

In some stents, the compressible and flexible properties that assist in stent delivery may also result in a stent that has a tendency to migrate from its originally deployed position. Stent migration affects many endoscopic stents including esophageal, duodenal, colonic, pancreatic, biliary and airway stents. It is thus desirable to provide a stent configuration that resists migration following deployment.

Commonly assigned US Patent Publication No. 20090098176 discloses medical devices with triggerable bioadhesives.

WO00/40278 A a stent graft which releases an agent which induces the in vivo adhesion of the stent to vessel walls to prevent migration of the stent graft and a coating which delays the onset of adhesion (e.g. PLGA).

Moreover, fully covered stents prevent tissue ingrowth and are easier to remove than bare or partially covered stents. However, these stents are even more prone to migration.

It is thus desirable to provide a stent configuration that resists migration following deployment.

Many techniques have been developed to prevent stent migration including adding barbs and flares to the stent itself or using clips or sutures to attach the stent to the vessel wall.

There remains a need in the art for an improved stent that is resistant to migration.

### SUMMARY OF THE INVENTION

In one embodiment, the present invention relates to a stent, the stent having an inner surface and an outer surface, at least a portion of the outer surface of the stent including a tacky biocompatible coating comprising a tacky silicone.

In another embodiment, the present invention relates to a stent, the stent having an inner surface and an outer surface, at least a portion of the outer surface of the stent including a tacky biocompatible coating comprising a tacky polymer material having a peel adhesion of 7.78 to 19.69 grams per cm (20 to 50 grams per inch), the tacky polymer having a tackiness that is not compromised by the presence of moisture.

In another embodiment, the present invention relates to a method of delivering a stent to a body lumen, the method including depositing a tacky biocompatible polymer material at a treatment site in a body lumen, delivering the stent to the treatment site and deploying the stent at the treatment site, wherein the tacky biocompatible polymer material hinders migration of the stent from the treatment site.

These and other aspects, embodiments and advantages of the present disclosure will become immediately apparent to those of ordinary skill in the art upon review of the Detailed Description and Claims to follow.

### BRIEF DESCRIPTION OF THE DRAWINGS

FIG. 1 is a side view of an embodiment of a stent according to the invention.
FIG. 2 is a cross-sectional view taken at 2 in FIG. 1 illustrating the tacky polymeric coating on the stent according to the invention.
FIG. 3 is a cross-sectional view of an alternative embodiment of a stent similar to that shown in FIG. 1 having the tacky polymeric coating and a hydrophilic coating or biodegradable coating disposed on the tacky polymeric coating.
FIG. 4 is a cross-sectional view of a covered stent according to the invention.

### DETAILED DESCRIPTION OF THE INVENTION

The present invention is directed to stents having a tacky coating thereon to prevent stent migration and to methods of using a tacky polymeric material in a patient's body lumen to prevent stent migration from a treatment site.

The term "tacky" is a well know term in the adhesives art. As used herein, the term "tacky" shall refer to a material that retains a sticky or slightly sticky feel to the touch. These materials can also be referred to as pressure sensitive polymer materials. The tacky materials employed herein can have peel strengths from 7.78 grams/cm to 393.7 grams/cm (20 grams/inch to 1000 grams/inch) as measured per ASTM D3330 Standard Test Method for Peel Adhesion of Pressure-Sensitive Tape, suitably 7.78 grams/cm to 196.85 grams/cm (20 grams/inch to 500 grams/inch), and more suitably 7.78 grams/cm to 39.37 grams/cm (20 grams/inch to 100 grams/inch). In some embodiments, the tacky materials employed herein have a low peel strength of 7.78 grams/cm to 19.69 grams/cm (20 grams/inch to 50 grams/inch).

The tacky polymeric materials employed herein are selected to as to provide gentle adhesion to human tissue. However, the adhesion is not permanent and the materials can be readily removed when desired.

It is also desirable that the tackiness or adhesion of the tacky polymeric material is not compromised upon exposure to moisture such as would be the case upon insertion in a patient's body.

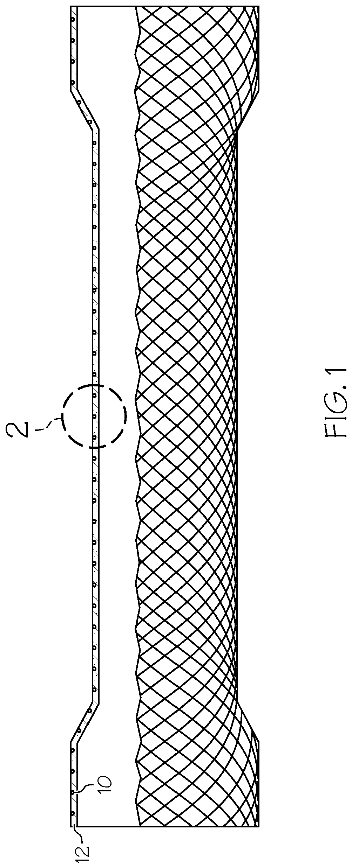

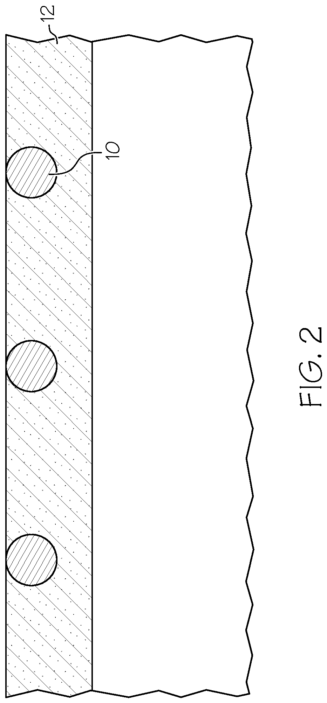

Turning now to the drawings, FIG. 1 is a side view of one embodiment of a stent on which the coatings according to the invention be employed. In this embodiment, stent 10 is a self-expanding stent formed of a shape memory metal such as nitinol having a silicone covering. The stent has a braided wire construction. In this embodiment, stent 10 is shown having a silicone covering 12. Stent 10 is disposed on silicone covering 12 and is partially embedded therein. FIG. 2 is a partial cross-sectional view of the stent taken at section 2 in FIG. 1. Stents of this type are described in commonly assigned US Patent Publication Nos. 2006/0276887 and 2008/0009934.

While in the embodiment shown in FIGS. 1 and 2, the stent is formed from nitinol, stents may be constructed of any suitable stent material including, but not limited to stainless steel, Elgiloy, nickel, titanium, nitinol, shape memory polymers, other polymeric materials, etc.

Any stent can have a covering and the coverings are thus not limited to nitinol stents. Moreover, the stent need not be covered whatsoever, may be partially covered or may be fully covered.

Other suitable covering materials can be employed as well. Examples of other suitable covering materials include, but are not limited to, polyethylene, polypropylene, polyvinyl chloride, polytetrafluoroethylene, including expanded polytetrafluoroethylene (ePTFE), fluorinated ethylene propylene, fluorinated ethylene propylene, polyvinyl acetate, polystyrene, poly(ethylene terephthalate), naphthalene, dicarboxylate derivatives, such as polyethylene naphthalate, polybutylene naphthalate, polytrimethylene naphthalate and trimethylenediol naphthalate, polyurethane, polyurea, polyamides, polyimides, polycarbonates, polyaldehydes, polyether ether ketone, natural rubbers, polyester copolymers, styrene-butadiene copolymers, polyethers, such as fully or partially halogenated polyethers, and copolymers and combinations thereof. See, for example, commonly assigned US Patent No. 8,114,147.

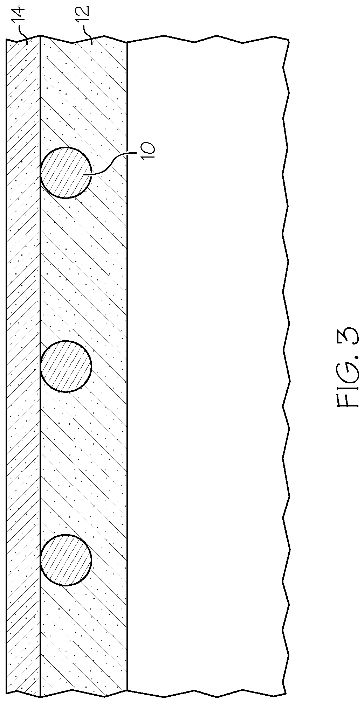

Stent 10 further has a tacky coating as shown in cross-section in FIG. 3. In this embodiment, tacky coating 14 comprises a tacky silicone.

Tacky or pressure sensitive silicone materials are commercially available from a variety of sources such as MED 6300 series of heat cured silicone materials and MED 6381 moisture cured silicone available from Nusil located in Santa Barbara, CA.

In some embodiments, the tacky silicone is a moisture cured silicone.

In some embodiments, the tacky silicone gel is a polydimethylsiloxane.

While tacky silicone is one desirable tacky polymeric material that may be employed herein, other tacky polymeric materials may be used as well including, but not limited to, styrenic block copolymers such as styrene-isobutylene-styrene (SIBS), styrene-ethylene/butylene-styrene (SEBS), styrene-ethylene/propylene-styrene (SEPS) and styrene-isoprene-styrene (SIS), acrylics, polyvinyl ether, polyurethanes, copolymers of ethylene such as ethylene vinyl acetate (EVA), etc.

The silicone gels have a similar feel to that of a hydrogel. However, the tackiness or adhesion of a hydrogel is compromised in the presence of moisture. Hydrogels are known to become "slippery" when wet, making them ideally suited for delivery of medical devices in a patient's body lumen where lubricity is desirable.

In some embodiments, a biocompatible dye is added to the tacky polymeric material to make it readily visible to a physician.

The tacky polymeric material may be applied to the entire outer surface of the stent, or to portions of the stent such as to the distal, proximal and central portions of the stent.

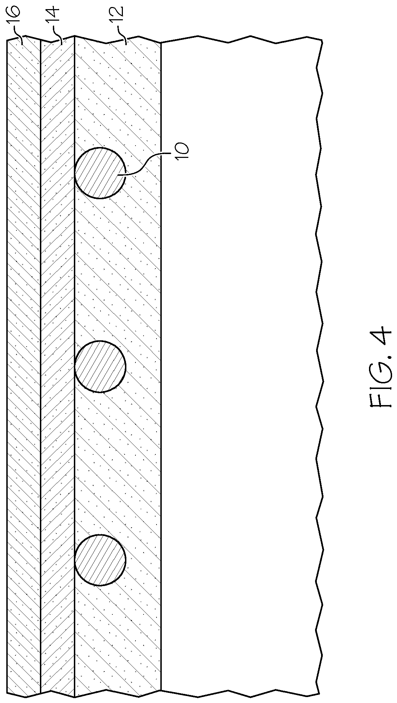

If the tacky polymeric material is applied to the stent, it may be desirable to dispose a hydrophilic or biodegradable coating over the tacky polymeric material to facilitate delivery through a patient's body lumen for a balloon expandable stent or to decrease the friction force between the outer tube of the delivery device and the stent for a self-expanding stent. FIG. 4 is a cross-sectional view of a stent 10 including a covering 12, a tacky polymeric coating 14 and a hydrophilic or biodegradable coating 16 disposed on the tacky polymeric coating 14. Once the stent is positioned and deployed at the treatment site, the biodegradable or hydrophilic coating will erode, exposing the underlying tacky polymeric coating which is now positioned between the patient's vessel wall and the stent in order to hinder stent migration.

Examples of suitable hydrogels include, but are not limited to, polyvinylpyrrolidone (PVP), poly(meth)acrylic acid and copolymers of (meth)acrylic acid, polyacrylate, chitosan, polyalkylene glycols such as polyethylene glycol (PEG) or polypropylene glycol, polyethylene glycol/dextran aldehyde, polyalkylene oxides such as polyethylene oxide and polypropylene oxide, polyvinyl esters such as polyvinyl acetate, polyhydroxyethyl methacrylate, polyvinyl alcohol, polyvinyl ether, and so forth. High molecular weight starches and carbohydrates may also be employed.

Hydrogel materials are disclosed in commonly assigned US Patent No. 5,693,034 to Buscemi et al.

Any suitable biodegradable material can be employed herein that does not form an adhesive layer. These biodegradable materials break down and lose their integrity *in vivo.* Examples of suitable biodegradable polymers include, but are not limited to, poly(amides) such as poly(amino acids) and poly(peptides), poly(esters) such as polylactide including poly(DL-lactide) and polyglycolide, and copolymers thereof such as polylactide-co-glycolide including poly(DL-lactide-co-glycolide), poly(L-lactide-co-glycolide), poly(caprolactone) and polylactide-co-caprolactone including poly(DL-lactide-co-caprolactone and poly(L-lactide-co-caprolactone), poly(anhydrides), poly(orthoesters), poly(carbonates) including tyrosine derived polycarbonates, polyhydroxyvalerate, polyhydroxybutyrate, polyhydroxybutyrate-co-valerate, and chemical derivatives thereof (substitutions, additions of chemical groups, for example, alkyl, alkylene, hydroxylations, oxidations, and other modifications routinely made by those skilled in the art), copolymers and mixtures thereof.

Therapeutic agents may be incorporated in the tacky polymeric material, the hydrophilic or biodegradable coating layer, or both.

Various therapeutic agents may be employed herein depending on the condition which is being treated. As used herein, the terms, "therapeutic agent", "drug", "pharmaceutically active agent", "pharmaceutically active material", "beneficial agent", "bioactive agent", and other related terms may be used interchangeably herein and include genetic therapeutic agents, non-genetic therapeutic agents and cells. A drug may be used singly or in combination with other drugs. Drugs include genetic materials, non-genetic materials, and cells.

A therapeutic agent may be a drug or other pharmaceutical product such as non-genetic agents, genetic agents, cellular material, etc. Some examples of suitable non-genetic therapeutic agents include but are not limited to: antithrombogenic agents such as heparin, heparin derivatives, vascular cell growth promoters, growth factor inhibitors, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof.

Other active agents include, but are not limited to, antineoplastic, antiproliferative, antimitotic, antiinflammatory, antiplatelet, anticoagulant, antifibrin, antiproliferative, antibiotic, antioxidant, and antiallergic substances as well as combinations thereof.

Examples of antineoplastic/antiproliferative/antimitotic agents include, but are not limited to, paclitaxel (e.g., TAXOL® by Bristol-Myers Squibb Co., Stamford, Conn.), the olimus family of drugs including sirolimus (rapamycin), biolimus (derivative of sirolimus), everolimus (derivative of sirolimus), zotarolimus (derivative of sirolimus) and tacrolimus, methotrexate, azathiprine, vincristine, vinblastine, 5-fluorouracil, doxorubicin hydrochloride, mitomycin, cisplatin, vinblastine, vincristine, epothilones, endostatin, angiostatin and thymidine kinase inhibitors. While the preventative and treatment properties of the foregoing therapeutic substances or agents are well-known to those of ordinary skill in the art, the substances or agents are provided by way of example and are not meant to be limiting. Other therapeutic substances are equally applicable for use with the disclosed methods and compositions. See commonly assigned U.S. Patent Application Nos. 2010/0087783, 2010/0069838, 2008/0071358 and 2008/0071350. See also commonly assigned U.S. Patent Application Nos. 2004/0215169 and 2009/0098176, and U.S. Patent No. 6,805,898.

Derivatives of many of the above mentioned compounds also exist which are employed as therapeutic agents and of course mixtures of therapeutic agents may also be employed.

For application, the therapeutic agent can be dissolved in a solvent or a cosolvent blend along with the bioadhesive or biodegradable polymer material.

Suitable solvents include, but are not limited to, dimethyl formamide (DMF), butyl acetate, ethyl acetate, tetrahydrofuran (THF), dichloromethane (DCM), acetone, acetonitrile, dimethyl sulfoxide (DMSO), butyl acetate, etc.

In other embodiments, the tacky polymeric material is delivered to the treatment site prior to delivery and deployment of the stent, for example via injection with a syringe, as in the esophagus, through or along the scope with a catheter, providing the viscosity is not too high for injection.

In some embodiments, the tacky polymeric material is a moisture cured polydimethylsiloxane which, once delivered to the treatment site, cures in the presence of moisture.

Once cured, the stent is then delivered and deployed at the treatment site. The tacky silicone material hinders stent migration. The stent can also be delivered before the tacky polymer is fully cured.

If the stent is a removable stent, the moisture cured polydimethylsiloxane can then be removed similar to removal of a dermal patch, such as with forceps.

If the viscosity of the tacky polymeric material is too high, it can be delivered as a patch and delivered with a scope. Suitably, the patch is about 1 cm in diameter, or delivery may involve the use of several smaller patches at more than one location to coincide with several locations along the stent, for example, distal, center and proximal locations of the stent.

If a patch is employed, it may be desirable to dispose a hydrophilic or biodegradable coating on the tacky polymeric material to facilitate delivery through a patient's body lumen.

If a hydrophilic coating is employed, water can be injected into the body lumen to facilitate dissolution/removal of the hydrophilic coating.

An alternative delivery technique is to employ a balloon to delivery the tacky polymeric material to the treatment site. Either patches or tubular members of the tacky polymeric material may be delivered in this manner. Multiple tubular members can also be delivered on a single balloon if desired.

For balloon delivery, it may also be desirable to dispose a hydrophilic polymer material on the tacky polymeric material to facilitate delivery through a patient's body lumen. The hydrophilic polymer may also be applied to the inner surface of the tacky polymeric material to prevent adhesion to the balloon. Dissolution/removal of the hydrophilic material can be facilitated by injecting water into the body lumen.

These techniques are most suitably employed with stents used in the gastrointestinal tract, but the techniques are not limited as such.

## Claims

1. A stent, the stent having an inner surface and an outer surface, at least a portion of the outer surface of the stent comprising:
a tacky biocompatible coating comprising a tacky silicone.

2. The stent of claim 1 wherein said tacky silicone has a tackiness that is not compromised by the presence of moisture.

3. The stent of claim 1 wherein said tacky silicone is pressure sensitive.

4. The stent of claim 1 wherein said tacky silicone is selected so as to provide adhesion to human tissue.

5. The stent of claim 1 comprising at least one second lubricious coating, the lubricious coating comprising a lubricious hydrophilic polymer material, the second lubricious coating is disposed over the tacky biocompatible coating.

6. The stent of claim 1 comprising at least one second biodegradable coating, the biodegradable coating is disposed over the tacky biocompatible coating.

7. The stent of claim 1 wherein said tacky silicone comprises polydimethylsiloxane.

8. The stent of claim 1 wherein said tacky silicone has a peel adhesion of 7.78 to 393.7 grams per cm (20 to 1000 grams per inch).

9. The stent of claim 1 wherein said tacky silicone has a peel adhesion of 7.78 to 39.37 grams per cm (20 to 100 grams per inch).

10. The stent of claim 1 wherein said biocompatible coating comprises a biocompatible dye.

11. The stent of claim 1 wherein said stent comprises a partial or full covering.

12. The stent of claim 11 wherein said covering comprises silicone.

13. The stent of claim 1 wherein said stent comprises a member selected from the group consisting of esophageal stents, pancreatic stents, duodenal stents, colonic stents, biliary stents and airway stents.

14. The stent of claim 2 wherein the tacky silicone has a peel adhesion of 7.78 grams per cm to 19.69 grams per cm (20 grams per inch to 50 grams per inch).

## Patentansprüche

1. Stent, wobei der Stent eine Innenfläche und eine Außenfläche hat und mindestens ein Abschnitt der Außenfläche des Stents aufweist:
eine klebrige biokompatible Beschichtung, die ein klebriges Silikon aufweist.

2. Stent nach Anspruch 1, wobei das klebrige Silikon eine Klebrigkeit hat, die durch das Vorhandensein von Feuchtigkeit nicht beeinträchtigt wird.

3. Stent nach Anspruch 1, wobei das klebrige Silikon druckempfindlich ist.

4. Stent nach Anspruch 1, wobei das klebrige Silikon so ausgewählt ist, dass es für Haftung an menschlichem Gewebe sorgt.

5. Stent nach Anspruch 1, der mindestens eine zweite gleitfähige Beschichtung aufweist, wobei die gleitfähige Beschichtung ein gleitfähiges hydrophiles Polymermaterial aufweist und die zweite gleitfähige Beschichtung über der klebrigen biokompatiblen Beschichtung angeordnet ist.

6. Stent nach Anspruch 1, der mindestens eine zweite biologisch abbaubare Beschichtung aufweist, wobei die biologisch abbaubare Beschichtung über der klebrigen biokompatiblen Beschichtung angeordnet ist.

7. Stent nach Anspruch 1, wobei das klebrige Silikon Polydimethylsiloxan aufweist.

8. Stent nach Anspruch 1, wobei das klebrige Silikon eine Klebekraft (peel adhesion) von 7,78 bis 393,7 Gramm pro cm (20 bis 1000 Gramm pro Inch) hat.

9. Stent nach Anspruch 1, wobei das klebrige Silikon eine Klebekraft (peel adhesion) von 7,78 bis 39,37 Gramm pro cm (20 bis 100 Gramm pro Inch) hat.

10. Stent nach Anspruch 1, wobei die biokompatible Beschichtung einen biokompatiblen Farbstoff aufweist.

11. Stent nach Anspruch 1, wobei der Stent eine teilweise oder vollständige Beschichtung aufweist.

12. Stent nach Anspruch 11, wobei die Beschichtung Silikon aufweist.

13. Stent nach Anspruch 1, wobei der Stent ein Teil aufweist, das aus der Gruppe ausgewählt ist, die aus Speiseröhren-Stents, Bauchspeicheldrüsen-Stents, Zwölffingerdarm-Stents, Dickdarm-Stents, Gallengang-Stents und Luftwege-Stents besteht.

14. Stent nach Anspruch 2, wobei das klebrige Silikon eine Klebekraft (peel adhesion) von 7,78 Gramm pro cm bis 19,69 Gramm pro cm (20 Gramm pro Inch bis 50 Gramm pro Inch) hat.

## Revendications

1. Endoprothèse, ladite endoprothèse présentant une surface intérieure et une surface extérieure, au moins une partie de la surface extérieure de ladite endoprothèse comprenant : un revêtement collant biocompatible comprenant un silicone collant.

2. Endoprothèse selon la revendication 1, où le silicone collant a une adhérence qui n'est pas affectée par la présence d'humidité.

3. Endoprothèse selon la revendication 1, où le silicone collant est sensible à la pression.

4. Endoprothèse selon la revendication 1, où le silicone collant est sélectionné de manière à présenter une adhérence sur un tissu humain.

5. Endoprothèse selon la revendication 1, comprenant au moins un deuxième revêtement lubrifiant, le revêtement lubrifiant comprenant un matériau polymère hydrophile lubrifiant, le deuxième revêtement lubrifiant étant disposé au-dessus du revêtement collant biocompatible.

6. Endoprothèse selon la revendication 1, comprenant au moins un deuxième revêtement biodégradable, ledit revêtement biodégradable étant disposé au-dessus du revêtement collant biocompatible.

7. Endoprothèse selon la revendication 1, où le silicone collant comprend du polydiméthylsiloxane.

8. Endoprothèse selon la revendication 1, où le silicone collant a une résistance au pelage comprise entre 7,78 et 393,7 grammes par cm (20 à 1000 grammes par pouce).

9. Endoprothèse selon la revendication 1, où le silicone collant a une résistance au pelage comprise entre 7,78 et 39,37 grammes par cm (20 à 100 grammes par pouce).

10. Endoprothèse selon la revendication 1, où le revêtement biocompatible comprend un colorant biocompatible.

11. Endoprothèse selon la revendication 1, où ladite endoprothèse présente une couverture partielle ou intégrale.

12. Endoprothèse selon la revendication 11, où la couverture comprend du silicone.

13. Endoprothèse selon la revendication 1, où ladite endoprothèse comprend un élément sélectionné dans le groupe comprenant des endoprothèses oesophagiennes, des endoprothèses pancréatiques, des endoprothèses duodénales, des endoprothèses coliques, des endoprothèses biliaires et des endoprothèses trachéobronchiques.

14. Endoprothèse selon la revendication 2, où le silicone collant a une résistance au pelage comprise entre 7,78 grammes par cm et 19,69 grammes par cm (20 grammes par pouce à 50 grammes par pouce).
